Europäisches Patentamt

European Patent Office

Office européen des brevets

(11) Veröffentlichungsnummer: **0 286 937 B1**

(12) **EUROPÄISCHE PATENTSCHRIFT**

(45) Veröffentlichungstag der Patentschrift: **29.01.92**

(21) Anmeldenummer: **88105369.8**

(22) Anmeldetag: **02.04.88**

(51) Int. Cl.5: **C07D 301/16, C07D 303/14**

(54) **Verfahren zur Herstellung epoxidierter Fettalkohole.**

(30) Priorität: **10.04.87 DE 3712183**

(43) Veröffentlichungstag der Anmeldung:
**19.10.88 Patentblatt 88/42**

(45) Bekanntmachung des Hinweises auf die
Patenterteilung:
**29.01.92 Patentblatt 92/05**

(84) Benannte Vertragsstaaten:
**AT BE CH DE ES FR GB GR IT LI NL SE**

(56) Entgegenhaltungen:
**EP-A- 0 032 990**
**EP-A- 0 033 110**
**WO-A-84/04920**
**US-A- 2 485 160**

(73) Patentinhaber: **Henkel Kommanditgesellschaft**
**auf Aktien**
**Postfach 1100 Henkelstrasse 67**
**W-4000 Düsseldorf-Holthausen(DE)**

(72) Erfinder: **Gruber, Bert, Dr.**
**Albert-Schlangen-Strasse 28**
**W-5012 Bedburg(DE)**

## Beschreibung

Die Erfindung betrifft ein Verfahren zur Herstellung von epoxidierten Fettalkoholen durch Umsetzung von ungesättigten Fettalkoholen mit 60-92 Gew.-% Wasserstoffperoxid enthaltenden wässrigen Lösungen und Ameisensäure in Abwesenheit von Katalysatoren bei 40-70° C, wobei man pro Mol zu epoxidierender Doppelbindung 1,1-3,5 Mol Wasserstoffperoxid und 0,05-1 Mol Ameisensäure einsetzt.

Das klassische Verfahren zur Herstellung von Epoxiden aus organischen Verbindungen mit mindestens einer olefinischen Doppelbindung ist die sogenannte Prileschajew-Reaktion, d.h. die Epoxidation mit Percarbonsäuren (vgl. D. Swern, Organic Peroxides, Vol. II, Wiley-Intersience, 1971, S. 355-533).

Bei der Verfahrensdurchführung geht man im allgemeinen nicht von den Percarbonsäuren aus, sondern man bringt die entsprechenden Carbonsäuren mit Wasserstoffperoxid zusammen, wobei sich Percarbonsäuren bilden, die in-situ mit den olefinischen Doppelbindungen reagieren. Als Carbonsäuren werden z.B. Ameisensäure, Essigsäure und höhere Homologe derselben verwendet. Häufig setzt man auch zur Beschleunigung der Reaktion Katalysatoren ein, z.B. Schwefelsäure und andere Mineralsäuren. Diese Katalysatoren beschleunigen zwar die Umsetzung, weisen jedoch den Nachteil auf, daß sie gleichzeitig Nebenreaktionen auslösen, welche im wesentlichen mit der Öffnung des Oxiranringes beginnen.

Für die Epoxidation von olefinisch ungesättigten organischen Verbindungen wird häufig in-situ aus Ameisensäure und Wasserstoffperoxid gebildete Perameisensäure eingesetzt. Dieses System hat den Vorteil, daß es für die Bildung der Percarbonsäure keines Katalysators bedarf. Aufgrund der Tatsache, daß Ameisensäure eine stärkere Säure darstellt als die übrigen für die in-situ-Epoxidation eingesetzten Carbonsäuren, ist hier eine erhöhte Tendenz zur unerwünschten Öffnung des Oxiranrings zu registrieren (vergl. D. Swern, loc. cit, S. 400-401).

Die in Abwesenheit von Katalysatoren mit in-situ gebildeter Perameisensäure durchgeführte Epoxidation von olefinischen Doppelbindungen wird bevorzugt bei der Herstellung von Epoxiden aus Olefine und ungesättigte Fettsäuren enthaltenden Triglyceriden angewandt (vergl. DE-C 30 02 861 und US-C 2 485 160).

Versucht man, die aus dem zitierten Stand der Technik bekannten, mit in-situ-Perameisensäure arbeitenden Epoxidationsverfahren auf die Herstellung von epoxidierten Fettalkoholen anzuwenden, so führt dies zu unbefriedigenden Ergebnissen. Neben den vorgenannten Nebenreaktionen ist hier eine unerwünschte Bildung von Ameisensäureestern zu beobachten, die dem epoxidierten Fettalkohol eine ungesättigte (hohe) Verseifungszahl verleihen.

Es wurde nun gefunden, daß man bei der in Abwesenheit von Katalysatoren durchgeführten Epoxidation von ungesättigten Fettalkoholen mit Hilfe von in-situ-Perameisensäure zu epoxidierten Fettalkoholen mit geringeren Gehalten an Nebenprodukten kommt, wenn man die Epoxidation in Gegenwart von 1-10 Gew.-%, bezogen auf Wasserstoffperoxidlösung, eines Puffers durchführt. Die erfindungsgemäß hergestellten epoxidierten Fettalkohole weisen neben einem hohen Anteil an Epoxidgruppen deutlich erniedrigte Verseifungszahlen auf.

Gegenstand der Erfindung ist demnach ein Verfahren zur Herstellung von epoxidierten Fettalkoholen durch Umsetzung von Fettalkoholen mit mindestens einer olefinischen Doppelbindung mit 60-92 Gew.-% Wasserstoffperoxid enthaltenden wässrigen Lösungen und Ameisensäure in Abwesenheit von Katalysatoren bei 40-70° C, wobei man pro Mol zu epoxidierender Doppelbindung 1,1-3,5 Mol Wasserstoffperoxid und 0,05-1 Mol Ameisensäure einsetzt, das dadurch gekennzeichnet ist, daß man die Epoxidation in Gegenwart von 1-10 Gew.-%, bezogen auf Wasserstoffperoxidlösung, eines Puffers durchführt.

Als Ausgangsmaterial eignen sich Fettalkohole mit 8 bis 22 Kohlenstoffatomen mit mindestens einer olefinischen Doppelbindung, insbesondere solche mit 16 bis 22 Kohlenstoffatomen, wie sie aus natürlichen Fetten und Ölen auf den bekannten Wegen der Fettalkoholsynthese zugänglich sind. Diese Fettalakohole werden in der Regel nicht als chemische Individuen, sondern als Gemische eingesetzt. Vorzugsweise dienen technische Oleylalkohole als Ausgangsmaterial, die neben dem in Mengen von etwa 70-90 Gew.-% vorhandenen einfach ungesättigten $C_{18}$-Fettalkohol auch mehrfach ungesättigte $C_{18}$-Fettalkohole und in untergeordneten Mengen gesättigte Fettalkohole enthalten. Es können jedoch auch ungesättigte Fettalkohole bzw. Fettalkoholfraktionen aus Raps- oder Fischöl epoxidiert werden, die einen erhöhten Anteil an $C_{20}$- bis $C_{22}$-Fettalkoholen enthalten.

Die für das erfindungsgemäße Verfahren erforderliche Ameisensäure kommt normalerweise in Form von wässrigen Lösungen mit höchstens 30 Gew.-% Wasser zum Einsatz.

Als Puffer zur Verwendung in dem Verfahren der Erfindung eignen sich allgemein Salze von schwach bis mäßig starken Säuren (pK_s gleich oder größer als 2), die gegenüber dem Epoxidationsmittel inert sind. Sie werden bevorzugt in einer Menge von 3-8 Gew.-%, bezogen auf Wasserstoffperoxidlösung, verwendet.

Bevorzugte Puffer für das Verfahren der Erfindung sind die, die aus der von Acetaten und Formiaten des Lithiums, Natriums, Kaliums, Calciums, Bariums and Zink, Hydrogenphosphaten des Natriums und Kaliums und Alkalioxalaten, insbesondere Natrium-und Kaliumoxalat, gebildeten Gruppe ausgewählt sind. Besonders bevorzugte Puffer sind diejenigen aus der von Natriumacetat, Kaliumacetat und sekundärem Natriumphosphat gebildeten Gruppe.

Bei der Durchführung der Epoxidation kann man ein Gemisch aus ungesättigtem Fettalkohol, Ameisensäure und Puffer vorlegen und Wasserstoffperoxid zugeben.

Gemäß einem weiteren bevorzugten Merkmal der Erfindung legt man den ungesättigten Fettalkohol und Ameisensäure vor und gibt die den Puffer enthaltende Wasserstoffperoxidlösung zu.

Die Erfindung wird im folgenden anhand von bevorzugten Ausführungsbeispielen näher erläutert.

Beispiel 1

135,0 g technischer Oleylalkohol (Ocenol® 90/95; Jodzahl (JZ) = 94) wurden mit 16,2 g 85%-iger Ameisensäure gemischt und nach Erhitzen auf 65 °C tropfenweise mit einer Lösung aus 3,65 g Natriumacetat in 73,0 g 70%-igem Wasserstoffperoxid versetzt. Nach einer Reaktionszeit von 2,5 h bei 65-70 ° wurde mit Wasser neutral gewaschen und das Epoxid im Vakuum getrocknet.

Der erhaltene epoxidierte Oleylalkohol hatte einen Epoxidsauerstoffgehalt von 4,2 % bei einer Jodzahl von 9 und einer Verseifungszahl (VZ) von 27.

Beispiele 2 bis 11

Die Umsetzung erfolgte analog zu den Bedingungen des Beispiels 1; in der nachfolgenden Tabelle sind die Einsatzmengen von Oleylalkohol, Ameisensäure, Wasserstoffperoxid und Puffern, die Reaktionszeiten sowie der Epoxidgehalt, die Jodzahlen (JZ) und Verseifungszahlen (VZ) der erhaltenen Epoxide zusammengefaßt. Weiterhin enthält die Tabelle einen Vergleichsversuch, bei dem ohne Puffer gearbeitet wurde. Das dabei erhaltene Produkt wies bei einem Epoxidgehalt von 3,5 % und einer Jodzahl von 3,5 eine Verseifungszahl von 50 auf, d.h. die Nebenproduktbildung wurde durch die erfindungsgemäße Verwendung von Puffern erheblich reduziert.

Tabelle 1

Zusammenstellung der durchgeführten Versuche

| Beispiel | Ocenol 90/95 [mol] | HCOOH (85%-ig) [mol] | $H_2O_2$ (70 %) [mol] | Puffer [% bez. auf $H_2O_2$-Lsg.] | Rkt.zeit [h] | % Epoxid | JZ | VZ |
|---|---|---|---|---|---|---|---|---|
| 1 | 1 | 0,6 | 3,0 | 5 % $CH_3COONa$ | 2,5 | 4,2 | 9 | 27 |
| 2 | 1 | 0,3 | 1,5 | 1 % " | 5,5 | 3,2 | 17 | 35 |
| 3 | 1 | 0,3 | 1,5 | 3 % " | 5,5 | 3,5 | 19 | 27 |
| 4 | 1 | 0,3 | 1,5 | 5 % " | 5,5 | 3,3 | 27 | 24 |
| 5 | 1 | 0,45 | 2,0 | 3 % " | 3,25 | 3,9 | 10 | 34 |
| 6 | 1 | 0,45 | 2,0 | 4 % " | 3,0 | 3,9 | 12 | 29 |
| 7 | 1 | 0,45 | 2,0 | 5 % " | 5,5 | 3,8 | 16 | 24 |
| 8 | 1 | 0,3 | 3,0 | 5 % " | 5,0 | 2,8 | 40 | 13 |
| 9 | 1 | 0,45 | 3,0 | 5 % " | 5,5 | 3,6 | 25 | 15 |
| 10 | 1 | 0,6 | 3,0 | 5 % $Na_2HPO_4$ | 1,5 | 4,1 | 6 | 34 |
| 11 | 1 | 0,6 | 3,0 | 5 % $CH_3COOK$ | 3,0 | 4,0 | 12 | 22 |
| Vergleich | 1 | 0,6 | 3,0 | - | 2,0 | 3,5 | 4 | 50 |

**Patentansprüche**

1.  Verfahren zur Herstellung von epoxidierten Fettalkoholen durch Umsetzung von Fettalkoholen mit mindestens einer olefinischen Doppelbindung mit 60-92 Gew.-% Wasserstoffperoxid enthaltenden wässrigen Lösungen und Ameisensäure in Abwesenheit von Katalysatoren bei 40-70°C, wobei man pro Mol zu epoxidierender Doppelbindung 1,1-3,5 Mol Wasserstoffperoxid und 0,05-1 Mol Ameisensäure einsetzt, dadurch gekennzeichnet, daß man die Epoxidation in Gegenwart von 1-10 Gew.-%, bezogen auf Wasserstoffperoxidlösung, eines Puffers durchführt.

2. Verfahren nach Anspruch 1, dadurch gekennzeichnet, daß man 3-8 Gew.-% des Puffers, bezogen auf Wasserstoffperoxidlösung, verwendet.

3. Verfahren nach Anspruch 1 oder 2, dadurch gekennzeichnet, daß man Puffer verwendet, die aus der von Acetaten und Formiaten des Lithiums, Natriums, Kaliums, Calciums, Bariums und Zink, Hydrogenphosphaten des Natriums und Kaliums und Alkalioxalaten, insbesondere Natrium- und Kaliumoxalat, gebildeten Gruppe ausgewählt sind.

4. Verfahren nach einem der Ansprüche 1 bis 3, dadurch gekennzeichnet, daß man Puffer verwendet, die aus der von Natriumacetat, Kaliumacetat und sekundärem Natriumphosphat gebildeten Gruppe ausgewählt sind.

5. Verfahren nach einem der Ansprüche 1 bis 4, dadurch gekennzeichnet, daß man den ungesättigten Fettalkohol und Ameisensäure vorlegt und die den Puffer enthaltende Wasserstoffperoxidlösung zugibt.

6. Verfahren nach einem der Ansprüche 1 bis 5, dadurch gekennzeichnet, daß man Fettalkohole mit 8 bis 22 Kohlenstoffatomen mit mindestens einer olefinischen Doppelbindung, herstellbar aus natürlichen Fetten und Ölen, epoxidiert.

7. Verfahren nach einem der Ansprüche 1 bis 6, dadurch gekennzeichnet, daß man Fettalkohole mit 16 bis 22 Kohlenstoffatomen mit mindestens einer olefinischen Doppelbindung, herstellbar aus natürlichen Fetten und Ölen, epoxidiert.

8. Verfahren nach einem der Ansprüche 1 bis 7, dadurch gekennzeichnet, daß man technische Oleylalkohole, enthaltend etwa 70-90 Gew.-% einfach ungesättigten $C_{18}$-Fettalkohol, Rest mehrfach ungesättigte $C_{18}$-Fettalkohole und kleinere Mengen gesättigter Fettalkohole, epoxidiert.

9. Verfahren nach einem der Ansprüche 1 bis 8, dadurch gekennzeichnet, daß man eine wässrige Ameisensäurelösung mit einem Wassergehalt von höchstens 30 Gew.-% verwendet.

## Claims

1. A process for the production of epoxidized fatty alcohols by reaction of fatty alcohols containing at least one olefinic double bond with aqueous solutions containing 60-92% by weight hydrogen peroxide and formic acid in the absence of catalysts at 40-70°C using 1.1-3.5 mol hydrogen peroxide and 0.05-1 mol formic acid per mol double bond to be epoxidized, characterized in that the epoxidation is carried out in the presence of 1-10% by weight, based on hydrogen peroxide solution, of a buffer.

2. A process as claimed in claim 1, characterized in that the buffer is used in a quantity of from 3 to 8% by weight, based on hydrogen peroxide solution.

3. A process as claimed in claim 1 or 2, characterized in that buffers selected from the group comprising acetates and formates of lithium, sodium, potassium, calcium, barium and zinc, hydrogen phosphates of sodium and potassium and alkali metal oxalates, more especially sodium and potassium oxalate, are used.

4. A process as claimed in any of claims 1 to 3, characterized in that buffers selected from the group comprising sodium acetate, potassium acetate and secondary sodium phosphate are used.

5. A process as claimed in any of claims 1 to 4, characterized in that the unsaturated fatty alcohol and the formic acid are initially introduced and the buffer-containing hydrogen peroxide solution subsequently added.

6. A process as claimed in any of claims 1 to 5, characterized in that $C_{8-22}$ fatty alcohols containing at least one olefinic double bond obtainable from natural fats and oils are epoxidized.

7. A process as claimed in any of claims 1 to 6, characterized in that $c_{16-22}$ fatty alcohols containing at least one olefinic double bond obtainable from natural fats and oils are epoxidized.

8. A process as claimed in any of claims 1 to 7, characterized in that technical oleyl alcohols containing approximately 70-90% by weight monounsaturated $C_{18}$ fatty alcohol and, for the rest, polyunsaturated $C_{18}$ fatty alcohols and relatively small quantities of saturated fatty alcohols are epoxidized.

9. A process as claimed in any of claims 1 to 8, characterized in that an aqueous formic acid solution containing at most 30% by weight water is used.

**Revendications**

1. Procédé de production d'alcools gras époxydés en faisant réagir des alcools gras ayant au moins une liaison double oléfinique avec une solution aqueuse contenant de 60 à 92% en poids de peroxyde d'hydrogène et avec de l'acide formique en l'absence de catalyseurs, à une température comprise entre 40 et 70°C en utilisant, par mole de liaison double à époxyder, 1,1 à 3,5 moles de peroxyde d'hydrogène et 0,05 à 1 mole d'acide formique, caractérisé en ce que l'époxydation est effectuée en présence de 1 à 10% en poids d'un tampon, rapportés à la solution de peroxyde d'hydrogène.

2. Procédé selon la revendication 1, caractérisé en ce qu'on utilise de 3 à 8% en poids de tampon, rapportés à la solution de peroxyde d'hydrogène.

3. Procédé selon la revendication 1 ou 2, caractérisé en ce qu'on utilise un tampon qui est choisi dans le groupe formé des acétates et des formiates de lithium, de sodium, de potassium, de calcium, de baryum et de zinc, des hydrogénophosphates de sodium et de potassium et des oxalates alcalins, et en particulier des oxalates de sodium et de potassium.

4. Procédé selon l'une des revendications 1 à 3, caractérisé en ce qu'on utilise un tampon qui est choisi dans le groupe formé de l'acétate de sodium, de l'acétate de potassium et du phosphate de sodium secondaire.

5. Procédé selon l'une des revendications 1 à 4, caractérisé en ce qu'on prépare l'alcool gras insaturé et l'acide formique et qu'on y ajoute la solution de peroxyde d'hydrogène contenant le tampon.

6. Procédé selon l'une des revendications 1 à 5, caractérisé en ce qu'on réalise l'époxydation d'alcools gras ayant de 8 à 22 atomes de carbone avec au moins une liaison double oléfinique, qui peuvent être obtenus au départ d'huiles et de graisses naturelles.

7. Procédé selon l'une des revendications 1 à 6, caractérisé en ce qu'on réalise l'époxydation d'alcools gras ayant de 16 à 22 atomes de carbone et au moins une liaison double oléfinique, qui peuvent être obtenus au départ d'huiles et de graisses naturelles.

8. Procédé selon l'une des revendications 1 à 7, caractérisé en ce qu'on réalise l'époxydation d'alcools oléyliques techniques contenant environ 70 à 90% en poids d'un alcool gras en $C_{18}$ insaturé une fois, le reste étant formé d'alcools gras en $C_{18}$ plusieurs fois insaturés et de faibles quantités d'alcools gras saturés.

9. Procédé selon l'une des revendications 1 à 8, caractérisé en ce qu'on utilise une solution aqueuse d'acide formique ayant une teneur en eau de 30% en poids au maximum.